# EUROPÄISCHE PATENTSCHRIFT

(11) **EP 0 768 316 B1**
(45) Veröffentlichungstag und Bekanntmachung des Hinweises auf die Patenterteilung: **25.02.1998**
(21) Anmeldenummer: 96115691.6
(22) Anmeldetag: 01.10.1996
(51) Int. Cl.: C07J 53/00, A61K 31/56

(54) **Steroide mit einer 14,15, Methylengruppe**
14,15-Methylene steroids
Stéroides 14,15-méthylènes

(30) Priorität: 10.10.1995 DE 19537626
(43) Veröffentlichungstag der Anmeldung: 16.04.1997
(73) Patentinhaber: Jenapharm GmbH & Co. KG, 07745 Jena (DE)
(72) Erfinder: Schwarz, Sigfrid, Prof. Dr., 07743 Jena (DE); Schubert, Gerd, Dr., 07743 Jena (DE); Siemann, Hans-Joachim, Dr., 07747 Jena (DE)

(56) Entgegenhaltungen:
- US-A- 4 231 946
- ARZNEIMITTEL FORSCHUNG DRUG RESEARCH., Bd. 30, Nr. 3, 1980, AULENDORF DE, Seiten 401-406, XP002018800 VON M. HÜBNER ET AL: "Eine Neue Klasse hichwirksamer Progestagene: 17.alpha.-CH2X-substituierte Gona-4,9(10)-diene"
- PHARMAZIE, Bd. 48, Nr. 7, Juli 1993, BERLIN DE, Seiten 541-545, XP002018801 M. OETTEL ET AL: "Das endokrinologische Profil von Metaboliten des Gestagens Dienogest"
- JOURNAL OF MEDICINAL CHEMISTRY, Bd. 29, Nr. 1, Januar 1986, WASHINGTON US, Seiten 54-60, XP002018802 JEAN-CHRISTOPHE DORE ET AL: "Correspondance Analysis Applied to Steroid Receptor Binding"
- "Grundlagen der allgemeinen und anorganischen Chemie"Ed.1968,pp347-348

## Beschreibung

Es werden neue Steroide mit einer 14,15-Methylengruppe der allgemeinen Formel I, 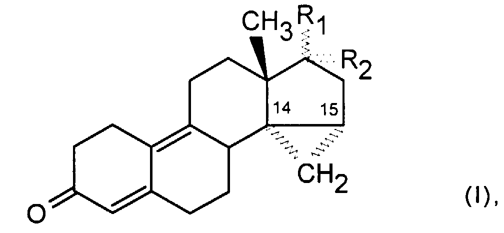 worin R₁ ein Wasserstoffatom, eine Hydroxygruppe, eine Alkyloxygruppe mit 1 bis 10 Kohlenstoffatomen oder einen Acyloxyrest
O-COR₃ bedeutet, worin R₃ 1 bis 10 Kohlenstoffatome umfaßt, eine Carbamoyloxygruppe O-CO-NHR₄ darstellt, worin R₄ für ein Wasserstoffatom, einen Alkyl- oder Arylrest steht oder eine Sulfamoyloxygruppe
O-SO₂ -NR₅ R₆ bedeutet, worin R₅ und R₆ unabhängig voneinander jeweils ein Wasserstoffatom oder eine Alkylgruppe mit 1 bis 5 Kohlenstoffatomen oder zusammen mit dem Stickstoffatom eine Pyrrolidino-, Piperidino- oder Morpholinogruppe darstellen,
R₂ unabhängig von R₁ eine Gruppierung -(CH₂)nCH₂X bedeutet, worin n = 0 bis 3 ist, X für ein Wasserstoffatom, einen Alkylrest mit 1 bis 10 Kohlenstoffatomen, eine Hydroxygruppe, eine Alkyloxygruppe mit 1 bis 10 Kohlenstoffatomen, eine Arylgruppe, ein Halogenatom oder ein Pseudohalogen, wie die Cyano- oder Azidogruppe steht,
R1 und R2 zusammen eine Oxogruppe oder einen Lactonring der allgemeinen Formel darstellt, 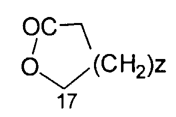 worin z die Werte 1 oder 2 annimmt und die 14,15-Methylengruppe entweder α- oder β-ständig angeordnet ist, einschließlich ihrer Herstellung beschrieben.

Verbindungen der allgemeinen Formel 1 haben hormonale Wirkungen. So weisen beispielsweise Verbindungen der allgemeinen Formel I, worin R₁ eine Hydroxygruppe, eine veresterte Hydroxygruppe oder eine Alkyloxygruppe bedeuten, R₂ unabhängig von R₁ ein Wasserstoffatom, eine Alkyloxyalkylgruppe oder eine Cyanomethylgruppe darstellen und die 14,15-Methylengruppe entweder α- oder β-ständig angeordnet sein kann, progestagene Wirkungen auf, wodurch diese Wirkstoffe in Kombination mit mindestens einem geeigneten Estrogen zur hormonalen Kontrazeption, in der Hormonreplacement-Therapie und zur Behandlung der Endometriose gestagenabhängiger Tumore geeignet sind.

Die erfindungsgemäßen Verbindungen der allgemeinen Formel I sind aus 3-Oxosteroiden der allgemeinen Formel II, 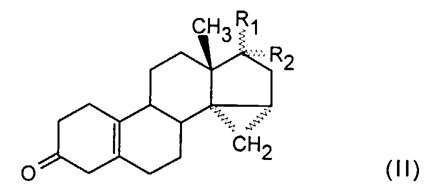 in der R₁ und R₂ die oben genannten Bedeutungen aufweisen und die 14,15-Methylengruppe entweder in α- oder β-Position angeordnet sein kann, in an sich bekannter Weise durch eine Bromierungs- / Dehydrobromierungsreaktion zugänglich.

3-Oxosteroide der allgemeinen Formel II, worin R₁ ein Wasserstoffatom, eine Hydroxygruppe, eine Alkyloxygruppe mit 1 bis 10 Kohlenstoffatomen oder einen Acyloxyrest O-COR₃ bedeutet, worin R₃ 1 bis 10 Kohlenstoffatome umfaßt,
eine Carbamoyloxygruppe O-CO-NHR₄ darstellt, worin R₄ für ein Wasserstoffatom, einen Alkyl- oder Arylrest steht oder eine Sulfamoyloxygruppe O-SO₂-NR₅R₆ bedeutet, worin R₅ und R₆ unabhängig voneinander jeweils ein Wasserstoffatom oder eine Alkylgruppe mit 1 bis 5 Kohlenstoffatomen oder zusammen mit dem Stickstoffatom eine Pyrrolidino-, Piperidino- oder Morpholinogruppe darstellen,
R₂ unabhängig Von R₁ eine Gruppierung -(CH₂)nCH₂X bedeutet, worin n 0 bis 3 ist, X für ein Wasserstoffatom, einen Alkylrest mit 1 bis 10 Kohlenstoffatomen,eine Hydroxygruppe, eine Alkyloxygruppe mit 1 bis 10 Kohlenstoffatomen oder eine Arylgruppe steht und die 14,15-Methylengruppe entweder α- oder β-ständig angeordnet ist, können ihrerseits aus aromatischen Steroiden der allgemeinen Formel III, worin R eine Alkylgruppe mit 1 bis 5 Kohlenstoffatomen darstellt, R₁ ein Wasserstoffatom, eine Hydroxygruppe, eine Alkyloxygruppe mit 1 bis 10 Kohlenstoffatomen bedeutet, R₂ unabhängig von R ₁ eine Gruppierung -(CH₂)nCH₂X darstellt, worin n 0 bis 3 ist, X für ein Wasserstoffatom, einen Alkylrest mit 1 bis 10 Kohlenstoffatomen,eine Hydroxygruppe, eine Alkyloxygruppe mit 1 bis 10 Kohlenstoffatomen oder eine Arylgruppe steht und die 14,15-Methylengruppe entweder α- oder β-ständig angeordnet ist, hergestellt werden.

Beispielsweise können Verbindungen der allgemeinen Formel III durch partielle Reduktion des aromatischen A-Ringes nach Birch mit Lithium in flüssigem Ammoniak und in Gegenwart eines Protonendonators in die Enolether IV umgewandelt werden, die nach Hydrolyse, beispielsweise mit Oxalsäure in Methanol und einer gegebenenfalls nachfolgenden Veresterung mit geeigneten Acyl- ,Carbamoyl- oder Sulfamoylchloriden bzw. einer Veretherung mit geeigneten Alkylhalogeniden 3-Oxosteroide der allgemeinen Formel II ergeben, in der R₁ ein Wasserstoffatom, eine Hydroxygruppe, eine Alkyloxygruppe mit 1 bis 10 Kohlenstoffatomen oder einen Acyloxyrest O-COR₃ bedeutet, worin R₃ 1 bis 10 Kohlenstoffatome umfaßt, eine Carbamoyloxygruppe O-CO-NHR₄ darstellt, worin R₄ für ein Wasserstoffatom, einen Alkyl- oder Arylrest steht oder eine Sulfamoyloxygruppe O-SO₂-NR₅R₆ bedeutet, worin R₅ undR₆ beispielsweise jeweils ein Wasserstoffatom oder eine Alkylgruppe mit 1 bis 5 Kohlenstoffatomen oder zusammen mit dem Stickstoffatom eine Pyrrolidino-, Piperidino- oder Morpholinogruppe darstellen, R₂ unabhängig von R₁ Wasserstoff oder eine Gruppierung -(CH₂)nCH₂X darstellt, worin n 0 bis 3 ist, X für ein Wasserstoffatom oder einen Alkylrest mit 1 bis 10 Kohlenstoffatomen, eine Hydroxygruppe, eine Alkyloxygruppe mit 1 bis 10 Kohlenstoffatomen oder eine Arylgruppe steht und die 14,15-Methylengruppe entweder α- oder β-ständig angeordnet ist.

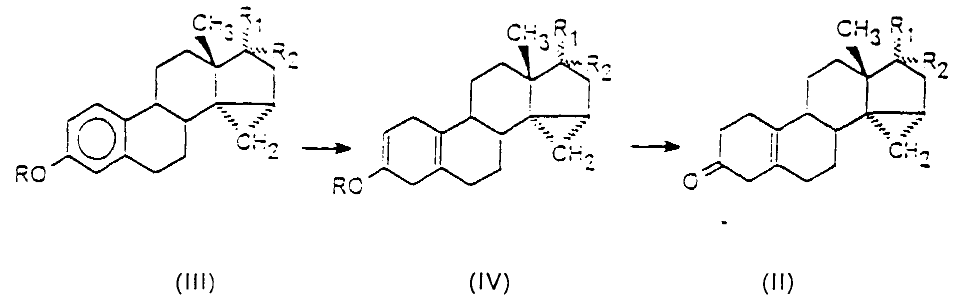

3-Oxosteroide der allgemeinen Formel II, worin R₁ und R₂ zusammen eine Oxogruppe darstellen und die 14,15-Methylengruppe entweder α- oder β-ständig angeordnet ist, können aus entsprechenden Verbindungen der allgemeinen Formel II, in der R₁ und R₂ eine Hydroxygruppe und ein Wasserstoffatom darstellen, durch Oxidation, beispielsweise nach Jones mit Chromschwefelsäure in Aceton, oder aus entsprechenden Enolethern der allgemeinen Formel IV, worin R₁ und R₂ ebenfalls eine Hydroxygruppe und ein Wasserstoffatom bedeuten, durch Oxidation, beispielsweise nach Oppenauer mit Cyclohexanon in Gegenwart von Aluminium-tert.butylat oder nach Collins mit Chrom-VI-oxid / Pyridin in Dichlormethan und nachfolgender Säurehydrolyse hergestellt werden.

3-Oxosteroide der allgemeinen Formel II, worin R₁ und R₂ zusammen einen Lactonring der allgemeinen Formel 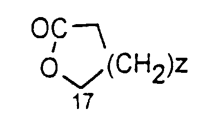 darstellt, worin z die Werte 1 oder 2 annimmt, und die 14,15-Methylengruppe entweder α- oder β-ständig angeordnet ist, werden aus 3-Oxosteroiden der allgemeinen Formel II, worin R₁ eine Hydroxygruppe darstellt und R₂ eine Gruppierung der allgemeinen Formel-CH2-(CH2)n-OH, worin n 2 oder 3 ist und die 14,15-Methylengruppe entweder α- oder β-ständig angeordnet ist, durch Oxidation der primären Hydroxylgruppe mit anschließender Lactonbildung hergestellt.

3-Oxosteroide der allgemeinen Formel II, worin R₁ eine Hydroxygruppe, eine Alkyloxygruppe mit 1 bis 10 Kohlenstoffatomen oder einen Acyloxyrest der allgemeinen Formel O-CO-R₃ bedeutet, worin R₃ 1 bis 10 Kohlenstoffatome umfaßt, eine Carbamoyloxygruppe der allgemeinen Formel O-CO-NHR₄ darstellt, worin R₄ beispielsweise für ein Wasserstoffatom, einen Alkyl- oder Arylrest steht oder eine Sulfamoyloxygruppe der allgemeinen Formel O-SO₂-NR₅R₆ bedeutet, worin R₅ und R₆ beispielsweise jeweils ein Wasserstoffatom oder eine Alkylgruppe mit 1 bis 5 Kohlenstoffatomen oder zusammen mit dem Stickstoffatom eine Pyrrolidino-, Piperidino- oder Morpholinogruppe darstellen, R₂ unabhängig von R₁ eine Gruppierung der allgemeinen Formel -(CH₂)nCH₂X darstellt, worin n 0 bis 3 ist, X für ein Wasserstoffatom, einen Alkylrest mit 1 bis 10 Kohlenstoffatomen, ein Halogenatom oder ein Pseudohalogen, wie die Cyano- oder Azidogruppe steht, bedeuten und die 14,15-Methylengruppe entweder α- oder β-ständig angeordnet ist, sind aus entsprechenden Enolethern der allgemeinen Formel IV, worin R₁ und R₂ zusammen eine Oxogruppe bilden, herstellbar.

So kann man diese Verbindungen beispielsweise in an sich bekannter Weise mit Grignard - Reagenzien oder anderen geeigneten Organometallverbindungen zur Reaktion bringen oder durch Carbonylolefinierung in die entsprechenden Oxirane der allgemeinen Formel IV überführen, worin R₁ und R₂ zusammen für eine Gruppe der Formel 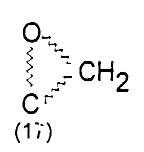 stehen, die dann mit Halogenwasserstoffen, Pseudohalogenwasserstoffen oder Grignardverbindungen eine Öffnung erfahren, wonach die Produkte einer Säurebehandlung unterzogen werden, wobei Verbindungen der allgemeinen Formel II entstehen, deren 17-Hydroxygruppe gegebenenfalls noch verestert oder verethert werden kann.

Ein weiterer Gegenstand der Erfindung sind pharmazeutische Zusammensetzungen, die als Wirkstoff neue Steroide mit einer 14,15-Methylengruppe der allgemeinen Formel I enthalten, wobei diese Zusammensetzungen gegebenfalls geeignete Hilfs-und Trägerstoffe enthalten.

Bevorzugte Darreichungsformen dieser Steroidwirkstoffe sind oral wirksame Formulierungen, transdermale Systeme oder Implantate.

Ferner können die erfindungsgemäßen pharmazeutischen Zusammensetzungen in Form von Mehrstufen- oder Kombinationspräparaten vorliegen.

Die Herstellung der erfindungsgemäßen Verbindungen der allgemeinen Formel I soll an den nachfolgenden Beispielen näher erläutert, jedoch nicht eingeschränkt werden.

### Beispiel 1

### Herstellung von 14α,15α-Methylenestra-4,9-dien-3-on-17α-ol aus 17α-Hydroxy-14α,15α-methylenestr-5(10)-en-3-on

17α-Hydroxy-14α,15α-methylenestr-5(10)-en-3-on (10 g) wurde in Pyridin (140 ml) gelöst. Zu der auf -10 °C abgekühlten und gerührten Lösung gab man dann portionsweise Pyridiniumbromidperbromid (11,2 g). Man rührte die Reaktionslösung ohne weitere Kühlung noch weitere 20 Stunden, kühlte dann wieder auf +5 °C ab und fügte wäßrige Schwefelsäure (1000 ml, 10 %ig) tropfenweise zu. Die entstandene Kristallsuspension wurde abfiltriert, die Kristalle wurden mit Wasser neutral gewaschen und getrocknet. Säulenchromatographie dieses Produktes an Kieselgel (Eluent Toluen / Ethylacetat / Dichlormethan 6 / 3 / 1) und Umkristallisation aus Methanol / Wasser ergaben die Titelverbindung. Fp. 113 - 115 °C: [α]_{D}²⁰ - 266 ° (c = 1, Chloroform).

### Herstellung des Ausgangsmaterials:

- Stufe a): 3-Methoxy-14α,15α-methylenestra-2,5(10)-dien-17α-ol aus 3-Methoxy-14α,15α-methylenestra-1,3,5(10)-trien-17α-ol
Die Reaktion ist unter Argonschutz und Ausschluß von Feuchtigkeit durchgeführt worden. 3-Methoxy-14α,15α-methylenestra-1,3,5(10)-trien-17α-ol (10 g) wurde in einem Gemisch aus trockenem, peroxidfreiem Tetrahydrofuran (130 ml) und Isopropanol (15,8 ml) gelöst. Diese Lösung tropfte man innerhalb von 5 Minuten zu flüssigem Ammoniak (124 ml) von -50 °C. Zu der entstandenen Suspension gab man dann Natrium (6,0 g, in kleinen Stücken) so zu, daß eine gleichbleibende Blaufärbung der Reaktionslö sung und eine Reaktionstemperatur von - 50 °C gewährleistet war. Nach etwa 3 Stunden war die Reduktion beendet (DC). Die Lösung wurde mit Ammoniumchlorid (5,0 g) zersetzt, das Ammoniak durch Erwärmen der Reak tionslösung (+ 30 °C) abgetrieben und das Tetrahydrofuran im Vakuumrotationsverdampfer weitgehend abdestilliert. Nach Zugabe von Wasser (300 ml) erhielt man eine Kri stallsuspension, die filtriert wurde. Das Filtergut wurde mit Wasser neutral gewaschen und getrocknet.
Die Titelverbindung konnte ohne weitere Reinigung in die nächste Stufe eingesetzt werden.
Fp. 155 - 160 °C.
- Stufe b): 17α-Hydroxy-14α,15α-methylenestr-5(10)-en-3-on aus 3-Methoxy-14α,15α-methylenestra-2,5(10)-dien-17α-ol
Eine Lösung von 3-Methoxy-14α,15α-methylenestra-2,5(10)-dien-17α-ol (15 g) in Methanol (1,28 l) wurde mit einer Lösung von Oxalsäuredihydrat (19,9 g) in Wasser (253 ml) vermischt und bei Raumtemperatur gerührt. Nach einer Stunde fügte man Wasser (300 ml) zu und setzte das Rühren bei + 15 °C für eine Stunde fort. Nach dem Abfil trieren wurden die Kristalle mit Methanol / Wasser 1 : 1 neutralgewaschen und getrocknet. Die Umkristallisation aus Aceton ergab die Titelverbindung.
Fp. 192 - 217 °C (Umlagerung);
[α]_{D}²⁰ + 221 ° (c = 1; Chloroform).

### Beispiel 2

### Herstellung von 3-Oxo-14β,15β-methylenestra-4,9-dien-17β-yl-(N-phenyl)carbamat

3-Oxo-14β,15β-methylenestr-5(10)-en-17β-yl-(N-phenyl)carbamat (178 mg) wurde in Pyridin (3 ml) gelöst. Bei -20 °C wurde Pyridiniumbromidperbromid (171 mg) zugegeben. Anschließend rührte man 3 Stunden bei Raumtemperatur, verdünnte mit Eiswasser, neutralisierte mit verdünnter Schwefelsäure und saugte den Niederschlag ab. Das Rohprodukt wurde durch präparative Schichtchromatographie an Kieselgel PF₂₅₄ mit Laufmittelgemisch Toluen /Aceton 9 : 1 gereinigt. Man erhielt farblose Kristalle, die aus Aceton umkristallisiert wurden. Fp.: 250-252 °C;

### Herstellung des Ausgangsmaterials:

- Stufe a): 3-Methoxy-14β,15β-methylenestra-2,5(10)-dien-17β-yl-(N-phenyl)carbamat
3-Methoxy-14β,15β-methylenestra-1,3,5(10)-trien-17β-(N-phenyl)carbamat (1,91 g) wurde in Tetrahydrofuran (15 ml) abs. gelöst, mit Isopropanol (4 ml) versetzt und unter Argonschutz zu 30 ml flüssigem Ammoniak zuge tropft. Nach Zugabe von weiterem Tetrahydrofuran (30 ml) gab man portionsweise Natrium zu, bis eine bleibende blaue Lösung vorlag. Nach 2,5 Stunden wurde mit Ammo niumchlorid zersetzt, das Ammoniak abgedampft, das Tetrahydrofuran unter Vakuum weitgehend eingeengt und mit wäßriger Ammoniumchlorid-Lösung ein Rohprodukt gefällt, das abgesaugt und getrocknet wurde.
Die Umkristallisation aus Methanol lieferte 3-Methoxy-14β,15β-methylenestra-2,5(10)-dien-17β-yl-(N-phenyl) carbamat als farblose Kristalle vom Fp. 179-186 °C.
- Stufe b): 3-Oxo-14β,15β-methylenestr-5(10)-en-17β-yl-(N-phenyl) carbamat
3-Methoxy-14β,15β-methylenestra-2,5(10)-dien-17β-yl-(N-phenyl)carbamat (576 mg) wurde in Dichlormethan (7 ml) gelöst und die Lösung mit tert. Butanol (25 ml) versetzt. Unter Rührung wurde Perchlorsäure (0,03 ml) in Wasser (7,5 ml) zugegeben. Nach 5 Stunden wurde mit wäßriger Natriumhydrogencarbonat-Lösung auf p_{H} 8 eingestellt, die Suspension mit Dichlormethan extrahiert, der Extrakt neu tral gewaschen, über Natriumsulfat getrocknet und unter Vakuum eingeengt. Man kristallisierte das 3-Oxo-14β,15β-methylenestr-5(10)-en-17β-yl-(N-phenyl)carbamat aus Aceton um; Fp: 201-215 °C (Zersetzung).

## Patentansprüche

1. Steroide mit einer 14,15-Methylengruppe der allgemeinen Formel I 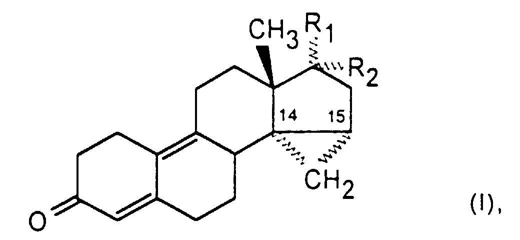 worin
R₁ ein Wasserstoffatom, eine Hydroxygruppe, eine Alkyloxygruppe mit 1 bis 10 Kohlenstoffatomen oder einen Acyloxyrest O-COR₃ bedeutet,
worin R₃ 1 bis 10 Kohlenstoffatome umfaßt,
eine Carbamoyloxygruppe O-CO-NHR₄ darstellt,
worin R₄ für ein Wasserstoffatom, einen Alkyl- oder Arylrest steht,
oder eine Sulfamoyloxygruppe O-SO₂-NR₅R₆ bedeutet,
worin R₅ und R₆ unabhängig voneinander jeweils ein Wasserstoffatom oder eine Alkylgruppe mit 1 bis 5 Kohlenstoffatomen
oder zusammen mit dem Stickstoffatom eine Pyrrolidino-, Piperidino- oder Morpholinogruppe darstellen,
R₂ unabhängig von R₁ Wasserstoff oder eine Gruppierung -(CH₂)nCH₂X bedeutet,
worin n = 0 bis 3 ist,
X für ein Wasserstoffatom, einen Alkylrest mit 1 bis 10 Kohlenstoffatomen, eine Hydroxygruppe, eine Alkyloxygruppe mit 1 bis 10 Kohlenstoffatomen, eine Arylgruppe, ein Halogenatom oder ein Pseudohalogen, wie die Cyano- oder Azidogruppe,
steht,
oder
R₁ und R ₂ zusammen eine Oxogruppe oder einen Lactonring der allgemeinen Formel 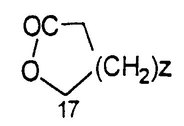
darstellt,
worin z die Werte 1 oder 2 annimmt und die 14,15-Methylengruppe entweder α- oder β-ständig angeordnet ist.

2. Steroide mit einer 14,15-Methylengruppe nach Anspruch 1, nämlich
17α-Hydroxy-14α,15α-methylenestra-4,9-dien-3-on,
17β-Hydroxy-14α,15α-methylenestra-4,9-dien-3-on,
17α-Hydroxy-14β,15β-methylenestra-4,9-dien-3-on,
17β-Hydroxy-14β,15β-methylenestra-4,9-dien-3-on,
14α,15α-Methylenestra-4,9-dien-3,17-dion,
14β,15β-Methylenestra-4,9-dien-3,17-dion,
17α-Cyanomethyl-17β-hydroxy-14α,15α-methyenestra-4,9-dien-3-on,
17α-Cyanomethyl-17β-hydroxy-14β,15β-methylenestra-4,9-dien-3-on,
17β-Hydroxy-17α-methyloxymethyl-14α,15α-methylenestra-4,9-dien-3-on,
17β-Methyloxy-17α-methyloxymethyl-14α,15α-methylenestra-4,9-dien-3-on,
17β-Hydroxy-17α-methyloxymethyl-14β,15β-methylenestra-4,9-dien-3-on,
17β-Methyloxy-17α-methyloxymethyl-14β,15β-methylenestra-4,9-dien-3-on
17β-Methyloxy-14β,15β-methylenestra-4,9-dien-3-on
14α, 15α-Methylen-3-oxo-17α-pregna-4,9-dien-21,17-carbolacton
17α-Azidomethyl-17β-hydroxy-14β,15β-methylenestra-4,9-dien-3-on
3-Oxo-14β,15β-methylenestra-4,9-dien-17β-yl - (N-phenyl) carbamat.

3. Verfahren zur Herstellung von Steroiden mit einer 14,15-Methylengruppe nach den Ansprüchen 1 oder 2,
dadurch gekennzeichnet,
daß man 3-Oxosteroide der allgemeinen Formel II, 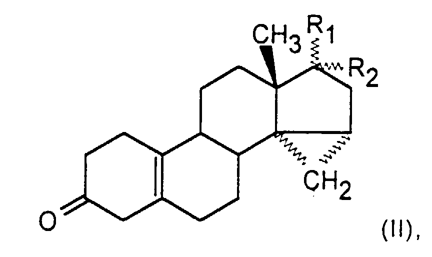 worin
R1 und R2 die im Anspruch 1 genannten Bedeutungen aufweisen
und die 14,15-Methylengruppe entweder α- oder β-ständig angeordnet ist,
in an sich bekannter Weise einer Bromierungs- und Dehydrobromierungsreaktion unterwirft und das Endprodukt gegebenenfalls verestert oder verethert.

4. Pharmazeutische Zusammensetzungen
enthaltend mindestens ein Steroid mit einer 14,15-Methylengruppe nach Anspruch 1 oder 2 gegebenenfalls zusammen mit pharmazeutisch verträglichen Hilfs- und Trägerstoffen.

## Claims

1. Steroids comprising a 14,15-methylene group of the general formula I 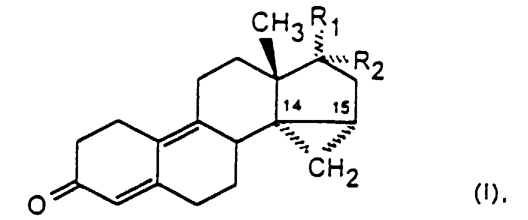 in which
R₁ means a hydrogen atom, a hydroxy group, an alkyloxy group with from 1 to 10 carbon atoms or an acyloxy residue O-COR₃,
in which R₃ comprises from 1 to 10 carbon atoms,
or represents a carbamoyloxy group O-CO-NHR₄,
in which R₄ denotes a hydrogen atom or an alkyl or aryl residue,
or a sulphamoyloxy group O-SO₂-NR₅R₆,
in which R₅ and R₆ each, independently of one another, represent a hydrogen atom or an alkyl group with from 1 to 5 carbon atoms or, together with the nitrogen atom, represent a pyrrolidino, piperidino or morpholino group,
R₂, independently of R₁, means hydrogen or a -(CH₂)nCH₂X grouping,
in which n = 0 to 3,
X denotes a hydrogen atom, an alkyl residue with from 1 to 10 carbon atoms, a hydroxy group, an alkyloxy group with from 1 to 10 carbon atoms, an aryl group, a halogen atom or a pseudohalogen, such as the cyano or azido group,
or
R₁ and R₂ together represent an oxo group or a lactone ring of the general formula 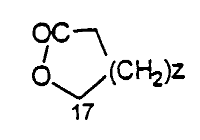
in which z assumes the values 1 or 2 and the 14,15-methylene group is located in the α or β position.

2. Steroids comprising a 14,15-methylene group according to claim 1, namely
17α-hydroxy-14α,15α-methyleneoestra-4,9-dien-3-one,
17β-hydroxy-14α,15α-methyleneoestra-4,9-dien-3-one,
17α-hydroxy-14β,15β-methyleneoestra-4,9-dien-3-one,
17β-hydroxy-14β,15β-methyleneoestra-4,9-dien-3-one,
14α,15α-methyleneoestra-4,9-dien-3,17-one,
14β,15β-methyleneoestra-4,9-dien-3,17-one,
17α-cyanomethyl-17β-hydroxy-14α,15α-methyleneoestra-4,9-dien-3-one,
17α-cyanomethyl-17β-hydroxy-14β,15β-methyleneoestra-4,9-dien-3-one,
17β-hydroxy-17α-methyloxymethyl-14α,15α-methyleneoestra-4,9-dien-3-one,
17β-methyloxy-17α-methyloxymethyl-14α,15α-methyleneoestra-4,9-dien-3-one,
17β-hydroxy-17α-methyloxymethyl-14β,15β-methyleneoestra-4,9-dien-3-one,
17β-methyloxy-17α-methyloxymethyl-14β,15β-methyleneoestra-4,9-dien-3-one,
17β-methyloxy-14β,15β-methyleneoestra-4,9-dien-3-one, 14α,15α-methylene-3-oxo-17α-pregna-4,9-dien-21,17-carbolactone
17α-azidomethyl-17β-hydroxy-14β,15β-methyleneoestra-4,9-dien-3-one
3-oxo-14β,15β-methyleneoestra-4,9-dien-17β-yl - (N-phenyl) carbamate.

3. A method of producing steroids comprising a 14,15 methylene group according to claim 1 or claim 2, characterised in that 3-oxosteroids of the general formula II 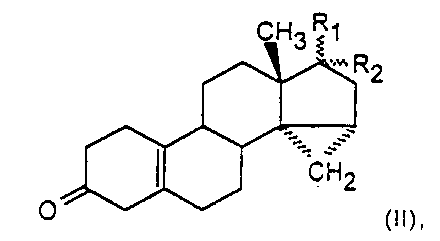 in which
R1 and R2 have meanings as given in claim 1 and the 14,15 methylene group is located in either the α or β position, is subjected in a manner known per se to a brominatio and dehydrobromination reaction and the end product i optionally esterified or etherified.

4. Pharmaceutical compositions containing at least one steroid comprising a 14,15 methylene group according to claim 1 or claim 2, optionally together with pharmaceutically acceptable auxiliary substances and carriers.

## Revendications

1. Stéroïde avec un groupe 14,15-méthylène de la formule générale I 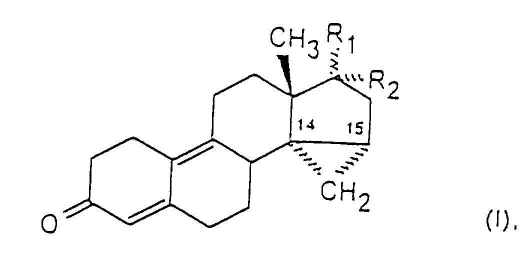 où
R₁ est un atome d'hydrogène, un groupe hydroxy, un groupe alkyloxy ayant 1 à 10 atomes de carbone ou bien un fragment acyloxy O-COR₃,
où R₃ contient 1 à 10 atomes de carbone, représente un groupe carbomoyloxy O-CO-NHR₄,
où R₄ indique un atome d'hydrogène, un résidu alkyle ou aryle,
ou bien un groupe sulfamoyloxy O-SO₂-NR₅R₆,
où R₅ et R₆, indépendamment l'un de l'autre représentent un atome d'hydrogène ou bien un groupe alkyle ayant 1 à 5 atomes de carbone,
ou bien ensemble avec l'atome d'azote, ils forment un groupe pyrrolidino, pipéridino ou morpholino
R₂, indépendamment de R₁, indique l'hydrogène ou un groupement -(CH₂)nCH₂X où n = 0 à 3,
X indique un atome d'hydrogène, un résidu alkyle avec 1 à 10 atomes de carbone, un groupe hydroxy, un groupe alkyle avec 1 à 10 atomes de carbone, un groupe aryle, un atome d'halogène ou un pseudo-halogène, comme le groupe cyano ou azido,
ou
R₁ et R₂, forment ensemble un groupe oxo ou bien un cycle de lactone de la formule générale 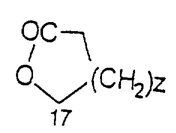
où Z prend la valeur de 1 ou 2 et le groupe 14, 15-méthylène est agencé en position α ou β.

2. Stéroïde avec un groupe 14, 15-méthylène selon la revendication 1,
c'est--à-dire
17α-hydroxy-14α,15α-méthylèneoestra-4,9-dièn-3-one,
17β-hydroxy-14α, 15α-méthylèneoestra-4,9-dièn-3-one,
17α-hydroxy-14β,15β-méthylèneoestra-4,9-dièn-3-one,
17β-hydroxy-14β,15β-méthylèneoestra-4,9-dièn-3-one,
14α,15α-méthylèneoestra-4,9-dièn-3, 17-dione,
14β,15β-méthylèneoestra-4,9-dièn-3, 17-dione,
17α-cyanomethyl-17β-hydroxy-14, 15-méthylèneoestra-4,9-dièn-3-one
17α-cyanomethyl-17β-hydroxy-14β,15β-méthylèneoestra-4,9-dièn-3-one
17β-hydroxy-17α-méthyloxyméthyl-14α,15α-méthylèneoestra-4,9-dièn-3-one,
17β-hydroxy-17α-méthyloxyméthyl-14α,15α-méthylèneoestra-4,9-dièn-3-one,
17β-hydroxy-17α-méthyloxyméthyl-14β,15β-méthylèneoestra-4,9-dièn-3-one,
17β-méthyloxy-17α-méthyloxyméthyl-14β,15β-méthylèneoestra-4,9-dièn-3-one,
17β-méthyloxy-14β,15β-méthylèneoestra-4,9-dièn-3-one, 14α,15α-méthylène-3-oxo-17α-prégna-4,9-dièn-21, 17-carbolactone,
17α-azidométhyl-17β-hydroxy-14β,15β-méthylèneoestra-4,9-dièn-3-one
3-oxo-14β,15β-méthylèneoestra-4,9-dièn-17β-yl(N-phényl) carbamate.

3. Procédé pour la production de stéroïdes avec un groupe 14,15-méthylène selon les revendications 1 ou 2,
caractérisé en ce que
on soumet un 3-oxostéroïde de la formule générale II 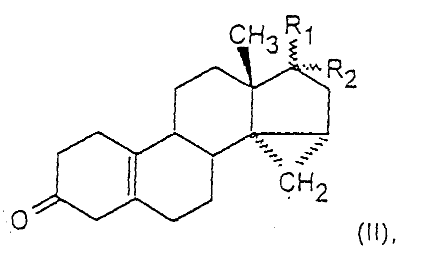 où
R1 et R2 ont les significations indiquées à la revendication 1
et le groupe 14, 15-méthylène est soit en position α ou β,
d'une manière connue, à une réaction de bromuration et de déshydrobromuration et le produit final est le cas échéant estérifié ou éthérifié.

4. Compositions pharmaceutiques contenant au moins un stéroïde avec un groupe 14, 15-méthylène selon la revendication 1 ou 2, le cas échéant avec des auxiliaires et supports pharmaceu tiquement acceptables.
